# EUROPEAN PATENT SPECIFICATION

(11) **EP 3 578 127 B1**
(45) Date of publication and mention of the grant of the patent: **14.02.2024**
(21) Application number: 18020391.1
(22) Date of filing: 16.08.2018
(51) Int. Cl.: A61B 34/30, A61B 90/57

(54) **A CANNULA SECURING ASSEMBLY FOR A MINIMALLY INVASIVE SURGICAL SYSTEM**
KANÜLENSICHERUNGSANORDNUNG FÜR EIN MINIMALINVASIVES CHIRURGISCHES SYSTEM
ENSEMBLE DE FIXATION DE CANULE POUR SYSTÈME CHIRURGICAL À INVASION MINIMALE

(30) Priority: 05.06.2018 IN 201811020960
(43) Date of publication of application: 11.12.2019
(73) Proprietor: Srivastava, Sudhir Prem, Gurugram, Haryana 122016 (IN)
(72) Inventor: Kapadia, Salman, 480661 Seoni, M.P (IN)
(74) Representative: Toro Gordillo, Ignacio Maria

(56) References cited:
- WO-A1-2015/010189
- WO-A1-2015/142814
- WO-A1-2017/120028
- US-A1- 2015 257 841

## Description

### TECHNICAL FIELD

The present disclosure relates generally to a minimally invasive surgical system, and more specifically to a cannula securing assembly for selectively engaging and disengaging a cannula in the minimally invasive surgical system. Moreover, the present disclosure relates to methods of operating the aforesaid minimally invasive surgical system.

### BACKGROUND

In this Background section, various aspects of art relevant to the present disclosure are described, to facilitate a better understanding of the present disclosure. Accordingly, it should be understood that such description is to be read in this light, and is not just admissions of prior art.

Robotically assisted surgical systems have been adopted worldwide to replace conventional surgical procedures to reduce an amount of extraneous tissue or tissues that may be damaged during surgical or diagnostic procedures, thereby reducing patient recovery time, patient discomfort, prolonged hospital tenure, and particularly deleterious side effects. In robotically assisted surgeries, a given surgeon typically operates a master controller at a surgeon console to capture seamlessly and transfer complex actions performed by the given surgeon giving the perception that the surgeon is directly articulating surgical tools to perform the surgery. The surgeon operating on the surgeon console may be located at a distance, for example remotely, from a surgical site or may be located within an operating theatre whereat the patient is being operated.

The robotically assisted surgeries have revolutionized the contemporary medical field, and one of the fastest growing sectors in medical device industry. However, a major challenge in robotically assisted surgeries is to ensure a high degree of safety and precision during the surgery. One key area of robotically assisted surgeries is the development of surgical robots for minimally invasive surgery. Over the last couple of decades, surgical robots have evolved in an exponential manner (namely, in respect of their precision and functionality) and has been a major area of innovation in the medical device industry.

The robotically assisted surgical systems comprise multiple robotic arms aiding in conducting robotic surgeries. In most robotically assisted surgeries, one or more small incisions are made in a given patient's body to provide an entry point for various surgical instruments and endoscopic devices. To guide the various surgical instruments and endoscopic devices inside the given patient's body during the robotically-assisted surgery, a cannula is firstly inserted through a point whereat the one or more small incisions are made. The cannula provides access to a body cavity of the given patient to perform various surgical procedures. Typically, the cannula is an elongate tube-like structure having one end with a pointed structure which is inside the body of the given patient during the surgical procedures. The other end of the cannula is attached onto a robotic arm assembly.

Performing surgeries with surgical instruments inside the patient body cavity through the cannula creates new challenges. One such challenge is the stability of the cannula during the manipulation of surgical instruments during the surgery. Another challenge is an ease of locking and unlocking the cannula from the surgical system.

Additionally, in surgical procedures, the cannula is docked to an assembly on the robotic arm assembly. Such an assembly has been convenient and effective for surgical procedures, but still requires further improvements in relation to effective cannula mounting, locking/unlocking of cannula, stability of the cannula during a surgical operation, and so forth.

WO 2015/142814 A1 describes a cannula mount for a surgical system may include a body having an aperture to receive a portion of a cannula. The cannula mount may further include a pivotable clamping arm to engage the portion of the cannula received in the aperture. The clamping arm may include a cam follower surface. The cannula mount may further include a block moveable between a first position and a second position. The block may include a cam surface. The cam surface of the block may engage the cam follower surface of the clamping arm in the first position to actuate the clamping arm to a closed position in which the clamping arm engages the portion of the cannula received in the aperture. The clamping arm may be permitted to move to an open position in which the clamping arm does not engage the cannula when the block is in the second position. In fact, WO2015/142814 A1 does describe the cannula being docked on the cannula mount on the robotic arm assembly, however still requires further improvements in relation to effective cannula mounting, locking/unlocking of cannula, stability of the cannula during a surgical operation, engagement of mount and the spring, and so forth.

WO 2015/010189 A1 describes a tool mount adaptor for interfacing a medical instrument with a medical insertion device are provided. The tool mount adaptor includes a collar for holding a medical instrument wherein the tool mount adaptor is releasably attachable to the medical insertion device. Cannula holder assemblies for a medical insertion device are also provided. The cannula holder assembly includes: (a) a cannula track; and (b) a cannula carriage slidably mounted on the cannula track comprising a cannula holder mount and a demobilizer. Medical insertion devices comprising the tool mount adaptors and/or cannula holder assemblies are also provided together with methods of using the medical insertion devices in diagnostic and/or therapeutic applications. However, WO 2015/010189 A1 does not describe a cannula securing arrangement where a compression mechanism or spring is used to engage or disengage a cannula.

WO 2017/120028 A1 describes a sheath for a surgical instrument may include a sleeve having a distal end and proximal end, and a retention mechanism at a proximal end portion of the sleeve. The retention mechanism may comprise a sleeve attachment portion configured to be attached to the sleeve at the proximal end portion, and a locking collar extending from the sleeve attachment portion, wherein the locking collar is configured to receive a connector portion of a surgical instrument. The locking collar further may include a locking feature movable between a first configuration, in which the sheath is moveable relative to the surgical instrument, and a second configuration, in which the locking feature is configured to engage with the connector portion to retain the sheath in position on the surgical instrument. However, WO 2017/120028 A1 does not describe a cannula securing arrangement where a compression mechanism or spring is used to engage or disengage a cannula.

In the light of aforementioned challenges, there is a need for an improved robotic surgical system with a cannula securing assembly for selectively engaging and disengaging a cannula.

### SUMMARY

The present disclosure seeks to provide an improved cannula securing assembly for selectively engaging and disengaging a cannula in a surgical system. The invention is limited by the scope of independent claim 1. Further embodiments are disclosed in the dependent claims.

In one aspect, an embodiment of the present disclosure provides a cannula securing assembly for selectively engaging and disengaging a cannula in a surgical system, the cannula securing assembly comprising:
- a housing including at least one aperture to receive a portion of the cannula;
- a locking plate including at least one aperture that engages in operation with the portion of cannula received in the at least one aperture of the housing and a releasing mechanism at one end of the locking plate, wherein the releasing mechanism is exposed when in operation on an exterior surface of the housing; and
- a compression mechanism affixed to a slot on an interior surface of the housing that engages when in operation with the locking plate,
characterized in that the compression mechanism biases when in operation the locking plate to facilitate engaging of the cannula with the cannula securing assembly, and the releasing mechanism disconnects when in operation the biasing of the locking plate to facilitate disengaging of the cannula with the cannula securing assembly.

Embodiments of the present disclosure substantially eliminate, or at least partially address, the aforementioned problems in the prior art, and enable more effective cannula mounting, locking/unlocking of the cannula, and enhance the stability of the cannula during a given surgical operation.

Optionally, the cannula securing assembly further comprises one or more sensors to measure force exerted on the cannula and to detect a presence of the cannula in the cannula securing assembly. More optionally, the one or more sensors are positioned on a Printed Circuit Board (PCB), wherein the PCB is affixed to an internal surface of the housing.

Optionally, the cannula securing assembly further comprises a retainer plate affixed to an interior surface of the housing, such that the retainer plate is fixed over the locking plate for facilitating a reciprocating movement of the locking plate.

Optionally, the compression mechanism is a flat spring.

Optionally, the cannula securing assembly is made of Aluminium.

Optionally, the cannula securing assembly receives when in operation a cannula sterile adaptor, such that the cannula sterile adaptor is positioned between the cannula securing assembly and cannula.

Optionally, the cannula sterile adaptor is connected to a drape such that the cannula sterile adaptor and the drape form a boundary between a sterile region and a non-sterile region.

In another aspect, an embodiment of the present disclosure provides a surgical system, comprising:
- a cannula; and
- a cannula securing assembly comprising:
   - a housing including at least one aperture to receive a portion of the cannula;
   - a locking plate including at least one aperture that engages in operation with the portion of cannula received in the at least one aperture of the housing and a releasing mechanism at one end of the locking plate, wherein the releasing mechanism is exposed when in operation on an exterior surface of the housing; and
   - a compression mechanism affixed to a slot on an interior surface of the housing to engage when in operation with the locking plate,
characterized in that the compression mechanism biases when in operation the locking plate to facilitate engaging of the cannula with the cannula securing assembly and the releasing mechanism disconnects when in operation the biasing of the locking plate to facilitate disengaging of the cannula with the cannula securing assembly.

Optionally, the surgical system further comprises a cannula sterile adaptor that is disposed between the cannula and cannula securing assembly, wherein the cannula sterile adaptor is connected to a drape such that the cannula sterile adaptor and the drape form a boundary between a sterile region and a non-sterile region.

Optionally, the cannula comprises a bowl section forming a proximal end of the cannula and a tube extending from the bowl section to a distal end of the cannula.

Optionally, the cannula further comprises an attachment portion, having locking prongs that engage or disengage the cannula wen in operation from the cannula securing assembly.

Optionally, the cannula securing assembly comprises a retainer plate affixed to an interior surface of the housing such that the retainer plate is fixed over the locking plate for facilitating a reciprocating movement of the locking plate.

Additional aspects, advantages, features and objects of the present disclosure would be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those skilled in the art will understand that the drawings are not to scale. Wherever possible, like elements have been indicated by identical numbers.

Embodiments of the present disclosure will now be described, by way of example only, with reference to the following diagrams wherein:
FIG. 1(a) is a schematic diagram of an operating room, illustrating multiple robotic arms of a robotic surgical system in accordance with an embodiment of the disclosure;
FIG. 1(b) is a schematic diagram of a surgeon console of the robotic surgical system in accordance with an embodiment of the disclosure;
FIG. 1(c) is a schematic diagram of a vision cart of the robotic surgical system in accordance with an embodiment of the disclosure;
FIG. 2(a) is an illustration of a perspective view of a tool interface assembly in accordance with an embodiment of the disclosure;
FIG. 2(b) is an illustration of an exploded view of the tool interface assembly in accordance with an embodiment of the disclosure;
FIG. 3(a) is an illustration of a perspective view of a cannula securing assembly attached to a cannula and a cannula sterile adaptor in accordance with an embodiment of the disclosure;
FIG. 3(b) is an illustration of an exploded view of the cannula securing assembly, cannula and cannula sterile adaptor in accordance with an embodiment of the disclosure;
FIG. 4 is an illustration of an exploded view of the cannula, the cannula sterile adaptor, and the cannula securing assembly in accordance with an embodiment of the disclosure;
FIG. 5(a) is an illustration of a side view of components of the cannula securing assembly in accordance with an embodiment of the disclosure;
FIG. 5(b) is an illustration of a top view of components of the cannula securing assembly in accordance with an embodiment of the disclosure; and
FIG. 6 is an illustration of a bottom view of a housing of the cannula securing assembly in accordance with an embodiment of the disclosure.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item at which the arrow is pointing.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

Throughout the detailed description, a convention employed is that in the appended drawings, like numerals denote like components.

Unless otherwise defined, all technical and scientific terms used herein have the same meaning as commonly understood by one of ordinary skill in the art. The apparatus, system, and examples provided herein are illustrative only and not intended to be limiting. The terms "a" and "an" herein do not denote a limitation of quantity, but rather denote the presence of at least one of the referenced item. Furthermore, the terms "sterile barrier" and "sterile adapter" denote the same meaning and may be used interchangeably throughout the description. Embodiments of the present disclosure will be described below in detail with reference to the accompanying drawings.

In one aspect, an embodiment of the present disclosure provides a cannula securing assembly for selectively engaging and disengaging a cannula in a surgical system, the cannula securing assembly comprising:
- a housing including at least one aperture to receive a portion of the cannula;
- a locking plate including at least one aperture configured to engage with the portion of cannula received in the at least one aperture of the housing and a releasing mechanism at one end of the locking plate, wherein the releasing mechanism is exposed when in operation on an exterior surface of the housing; and
- a compression mechanism affixed to a slot on an interior surface of the housing that engages when in operation with the locking plate,
characterized in that the compression mechanism biases when in operation the locking plate to facilitate engaging of the cannula with the cannula securing assembly and the releasing mechanism is configured for disconnecting the biasing of locking plate to facilitate disengaging of the cannula with the cannula securing assembly.

In another aspect, an embodiment of the present disclosure provides a surgical system, comprising:
- a cannula; and
- a cannula securing assembly comprising:
   - a housing including at least one aperture to receive a portion of the cannula;
   - a locking plate including at least one aperture configured to engage with the portion of cannula received in the at least one aperture of the housing and a releasing mechanism at one end of the locking plate, wherein the releasing mechanism is exposed when in operation on an exterior surface of the housing; and
   - a compression mechanism that is affixed when in operation to a slot on an interior surface of the housing and engages when in operation with the locking plate,
characterized in that the compression mechanism biases when in operation the locking plate to facilitate engaging of the cannula with the cannula securing assembly and the releasing mechanism disconnects when in operation the biasing of locking plate to facilitate disengaging of the cannula with the cannula securing assembly.

The present disclosure relates to a robotic surgical system for performing minimally invasive surgery. The robotic surgical system will generally involve the use of multiple robotic arms. One or more of the robotic arms will often support a surgical tool which may be articulated (such as jaws, scissors, graspers, needle holders, micro dissectors, staple appliers, tackers, suction/irrigation tools, clip appliers, or the like) or non-articulated (such as cutting blades, cautery probes, irrigators, catheters, suction orifices, or the like). One or more of the robotic arms will often be used to support one or more surgical image/ video capture devices such as an endoscope (which may be any of the variety of structures such as a laparoscope, an arthroscope, a hysteroscope, or the like), or optionally, some other imaging modality (such as ultrasound, fluoroscopy, magnetic resonance imaging, or the like).

In an embodiment, the robotic surgical system may have a plurality of robotic arms mounted around a patient cart; for example, the robotic surgical system may have four robotic arms. The number of robotics arms employed may vary depending upon a type of surgery to be performed or the robotic surgical system. The robotic arms, for example four robotic arms, may be mounted along the patient cart and may be arranged in various different manners but not limited to the robotic arms mounted on the patient cart or the robotic arms separately mounted on a movable means or the robotic arms mechanically and/ or operationally connected with each other or the robotic arms connected to a central body such that the robotic arms branch out of the central body.

In another embodiment, the surgeon console may aid the surgeon to operate remotely a given patient lying on the patient cart by controlling the robotic arms around the body of the given patient. The surgeon console may control when in operation the movement of surgical instruments while the instruments are inside the patient body. The surgeon console may comprise of at least an adjustable viewing apparatus, but not limited to 2D/ 3D monitors, wearable viewing apparatus and any combination thereof. The surgeon console may be equipped with multiple displays which would not only show 3D high definition (HD) endoscopic view of a surgical site at the patient cart but may also show additional information from various medical equipment's which the surgeon may use during the robotic surgery. Furthermore, the viewing apparatus may provide various modes of the robotic surgical system, but not limited to an identification and an number of robotic arms attached, current surgical instrument type attached, current instrument tool tip position, collision information along with medical data like ECG, ultrasound display, fluoroscopic images, CT, MRI information. The surgeon console may further comprise a mechanism for controlling the robotics arms but not limited to one or more hand controllers, one or more-foot controllers, a clutch mechanism, and in combination thereof. The hand controllers at the surgeon console are required to capture seamlessly and to transfer complex actions performed by surgeon giving the perception that the surgeon is directly articulating the surgical tools. The different controllers may require for different purpose during the surgery. In some embodiments, the hand controllers may be one or more manually-operated input devices, such as a joystick, exoskeletal glove, a powered and gravity-compensated manipulator, or the like. These hand controllers control teleoperated motors which, in turn, control the movement of the surgical instruments attached to the robotic arms. The surgeon may sit on a resting apparatus such as a chair, while controlling the surgeon console. The chair may be adjustable in respect of its height, elbow rest, and the like according to the ease of the surgeon and also various control mechanisms may be provided on the chair. Furthermore, the surgeon console may be at a single location inside an operation theatre or may be distributed at any other location in the hospital providing for connectivity to the robotics arms to be maintained.

In an embodiment, the vision cart may display when in operation the 2D and/or 3D view of the operation captured by an endoscope. The vision cart may be adjusted at various angles and heights depending upon the ease of view. The vison cart may have various functionality, but not limited to providing touch screen display, preview/recording/playback provisions, various inputs/ outputs means, 2D to 3D converters, and the like. The vision cart may include a vision system or displays that enables a spectator or other non-operating surgeons/assistants to view a surgical site from outside the patient's body. One of the robotics arms typically engage a camera that has a video-image-capture function (i.e., an endoscopic camera) for displaying the captured images on the vision cart. In some robotic surgical system configurations, the camera includes optics that transfer the images from the distal end of the camera to one or more imaging sensors (e.g., CCD or CMOS sensors) outside of the patient's body. Alternatively, the one or more imaging sensors may be positioned at the distal end of the camera, and the signals produced by the one or more sensors may be transmitted along a wire or wirelessly for processing and display on the vision cart.

In another embodiment, the tool interface assembly may be mounted on any of the robotic arm of the robotic surgical system. The tool interface assembly may be the main component for performing the robotic surgery on a patient.

The tool interface assembly may comprise of an Arm and Tool Interface (ATI) connector which facilitates the tool interface assembly to connect operationally with the robotic arm. The tool interface assembly may further comprise of an actuator assembly mounted on a guiding mechanism and capable of linearly moving along the guiding mechanism. The guiding mechanism may be a guide rail. Furthermore, the movement of the actuator assembly along the guide rail is controlled by the surgeon with the help of hand controllers on the surgeon console. A sterile adapter assembly is releasably mounted on the actuator assembly to separate a non-sterile part of the robotic arm from a sterile surgical tool assembly. A locking mechanism may be provided to releasably lock and unlock the sterile adapter assembly with the actuator assembly. The sterile adaptor assembly may detachably engage with the actuator assembly which drives and controls the sterile surgical instrument in a sterile field. In another embodiment, the sterile surgical tool assembly also may be releasably lock/ unlock or engages/disengages with the sterile adapter assembly by means of a push button.

In an embodiment, the sterile surgical tool assembly may include a shaft and end effectors. The end effectors may comprise a surgical instrument that is integrated with the end effector, or the end effector may include an attachment mechanism to attach a surgical instrument where the end effector may act as a pluggable device. The surgical instrument may be associated with one or more surgical tasks, such as a forcep, a needle driver, a shears, a bipolar cauterizer, a tissue stabilizer or retractor, a clip applier, an anastomosis device, an imaging device (e.g., an endoscope or ultrasound probe), and the like. Some surgical instruments further provide an articulated support (sometimes referred to as a "wrist") for the sterile surgical tool assembly such that the position and orientation of the surgical tool assembly may be manipulated with one or more mechanical degrees of freedom in relation to the instrument's shaft. Furthermore, the end effectors may include a functional mechanical degree of freedom, such as jaws that open or close, or a knife that translates along a path. The sterile surgical tool assembly may also contain stored (e.g., on a memory inside the instrument) information that may be permanent or may be updatable by the robotic surgical system.

In an embodiment, the cannula securing assembly may be affixed to the body of the tool interface assembly and may grip or secure when in operation the cannula such that cannula is stable while performing surgical operations. The cannula securing assembly may be affixed to a mount of the tool interface assembly by bolts. Alternatively, the cannula securing assembly may be riveted, screwed or a combination thereof to the mount of the tool interface assembly. According to a specific embodiment of the disclosure, opposite sides of the cannula securing assembly may be bolted on respective opposite sides of the mount of the tool interface assembly.

In another embodiment, the cannula securing assembly may be made of any suitable resilient material such as a metal or an alloy. The material for the cannula securing assembly can be optionally selected from a group consisting of Aluminium, steel, Iron, Nickel, Copper, Zinc, Tin, or any combination thereof. In accordance to a specific embodiment of the present disclosure, the cannula securing assembly is made of Aluminium. The cannula securing assembly may be painted or may have a protective coating such as an alloy coating. In accordance with an embodiment, the process of anodizing may be used to coat the cannula securing assembly, such as to form a protective coating of Aluminum Oxide on the surface of the cannula securing assembly. The cannula securing assembly may be of any suitable size that can be conveniently attached to the mount of the tool interface assembly without affecting ease of the surgical operation. The cannula securing assembly may be of a suitable thickness providing sufficient strength.

In yet another embodiment, the cannula securing assembly may be of any suitable shape (suitably similar to the mount of the tool interface assembly) such that the ease of affixing the cannula securing assembly is maintained. In accordance with an embodiment of the disclosure, the cannula securing assembly is substantially of a square shape, where a bottom end of the cannula securing assembly is substantially larger than a top end of the cannula securing assembly and the body of the cannula securing assembly is substantially tapered towards the top end of the cannula securing assembly.

Referring now about the cannula, the cannula may comprise a hollow body. The cannula is fixed to the shaft at a desired angle and precludes shifting, twisting or any axial movement of the shaft once received by the cannula. The cannula may be inserted through an opening in a patient's body to a surgical site. For example, a first end of cannula (explained later) may be inserted through an opening, such as, for example, an incision, natural orifice, or port, to a surgical site. In an exemplary embodiment, a surgical instrument may be inserted through the cannula to the surgical site. For example, an instrument may be inserted into a second end of cannula and advanced through the hollow body and the first end of the cannula to the surgical site.

Furthermore, the cannula may be made of any suitable resilient material such as a metal or an alloy. The material for the cannula can optionally be selected from a group consisting of Aluminium, steel, Iron, Nickel, Copper, Zinc, Tin, or any combination thereof. In accordance to a specific embodiment of the disclosure, the cannula is made of Aluminium. The cannula may be painted or may have a protective coating such as alloy coating. In accordance with an embodiment, the process of anodizing may be used to coat the cannula such as to form a protective coating of Aluminum Oxide on the surface of the cannula. The cannula may be of any suitable size that can be conveniently attached to the cannula securing assembly without affecting ease of the surgical operation. The cannula may be of a suitable thickness providing sufficient strength.

Furthermore, the cannula may be of any suitable shape such that the ease of affixing the cannula is maintained. In accordance with an embodiment of the disclosure, the cannula is a long elongate hollow tube attached to a bowl section.

In an embodiment, a cannula sterile adaptor may be mounted between the cannula securing assembly and the cannula, with the cannula sterile adaptor being connected or sealed (may be thermal sealing) to a drape such the cannula sterile adaptor and the drape may form a boundary between a sterile region and a non-sterile region. For better understanding, the part of cannula sterile adaptor that is connected or sealed to the surgical drape with a sterile region on the side of the drape facing cannula and the non-sterile region on the side of the drape facing cannula securing assembly.

In another embodiment, the cannula sterile adaptor may be made of a relatively rigid material that provides structural support for the cannula sterile adaptor when the cannula sterile adaptor is mounted to the cannula securing assembly, as well as for the cannula when the cannula is mounted to the cannula securing assembly. Furthermore, the relatively rigid material can be a smooth, low-friction material, which may facilitate alignment and insertion of cannula into cannula sterile adaptor by providing a low-friction surface over which attachment portions may easily slide. In addition, the cannula sterile adaptor may be designed (namely configured) to accommodate forces applied between cannula and the cannula securing assembly, such as clamping forces and body wall forces, when cannula is mounted to the cannula securing assembly.

In yet another embodiment, the cannula sterile adaptor may be optionally made of a plastics material, such as, for example, polycarbonate, acrylonitrile butadiene styrene (ABS), polycarbonate/ABS, polyurethane, and other plastics materials familiar to one of ordinary skill in the art. The low-friction surface may also assist with latching the cannula to the cannula securing assembly by facilitating sliding of cannula when a latching force supplied by the cannula securing assembly draws the cannula into a mounting position during latching. According to a specific embodiment, the cannula sterile adaptor may be treated with a lubricant to facilitate insertion and/or removal of the cannula. The lubricant may be, for example, a dry coating of polytetrafluoroethylene (PTFE) or other lubricant familiar to one of ordinary skill in the art that is applied to a surface of cannula sterile adaptor. According to a specific embodiment, the cannula sterile adaptor is a hat-like structure which encloses or sits over the top end of the cannula securing assembly.

In an embodiment, the cannula may include a bowl section forming a proximal end of the cannula, and a tube may extend from the bowl section to a distal end of the cannula. According to a specific embodiment, the tube may have a length L, the distal end and proximal end may have a diameter D, Di, and the bowl section having a circumference C, each of which may vary depending on a desired application of the cannula. Furthermore, in a specific embodiment, the tube may be straight, although the exemplary cannula embodiments described herein are not limited to a straight tube. For example, the tube may instead be a curved tube having a substantially curved longitudinal axis. The cannula may also comprise of an attachment portion which encloses the bowl section and may have a protruding end. The protruding end of the attachment portion may be provided with locking prongs which helps the attachment portion in engaging or disengaging from the cannula securing assembly.

In an embodiment, the cannula sterile adaptor may be connected to the cannula securing assembly, such as by inserting holding portions of the cannula sterile adaptor into respective apertures of the cannula securing assembly. According to a specific embodiment, a pair of holding portion and a pair of respective recesses are provided. The cannula sterile adaptor may function with one holding portion and one recess also. According to another specific embodiment, the shape of the holding portion corresponds to the shape of the recess of the cannula securing assembly such as to facilitate mounting of the cannula sterile adaptor in the cannula securing assembly.

In another embodiment, the cannula sterile adaptor may facilitate forming a boundary between a sterile region and a non-sterile region. For example, a surgical drape may be attached to the cannula sterile adaptor to separate a sterile side from a non-sterile side of the drape.

In an embodiment, the attachment portions of the cannula may be structured to fit inside openings of the cannula sterile adaptor so that the cannula remains on sterile side of the drape. Furthermore, when the cannula sterile adaptor has been connected to the cannula securing assembly, and attachment portions are inserted into the openings of the cannula sterile adaptor, the cannula may also be connected to the cannula securing assembly so that the cannula may be held by the cannula securing assembly during a surgical procedure. Optionally, the cannula securing assembly may function with one attachment portion and one opening also.

Alternatively, the attachment portion of the cannula may be structured to fit inside the recess of the cannula securing assembly in case the cannula sterile adaptor is not used in surgical operations.

According to an embodiment, the shape of the attachment portions corresponds to the shape of the openings of the cannula sterile adaptor such as to facilitate mounting of the cannula in the cannula sterile adaptor.

According to an exemplary embodiment, the attachment portions may include locking prongs, such as on one side of attachment portion, to assist with mounting the cannula to the cannula securing assembly. Locking prongs may be configured to facilitate mounting of the cannula to the cannula sterile adaptor and thereon to the cannula securing assembly. For example, an angle of locking prongs may be selected to facilitate an application of a large amount of force between the attachment portions, cannula sterile adaptor, and cannula securing assembly when cannula is mounted, but also facilitate release of the attachment portions. According to a specific embodiment, the locking prongs may have a protruding profile substantially orthogonal to the attachment portions.

In an embodiment, the housing may be a shell for enclosing the parts of the cannula securing assembly. The housing also includes an aperture to receive the holding portion of the cannula sterile adaptor having the attachment portion of the cannula contained therein. According to a specific embodiment, a pair of apertures is provided to receive a pair of holding portions. The disclosure may function with one recess and one holding portion also. According to an embodiment, the shape of the aperture corresponds to the shape of the holding portion of the cannula sterile adaptor such as to facilitate mounting of the cannula in the housing of the cannula securing assembly.

In another embodiment, the housing may be made of any suitable resilient material such as a metal or an alloy. Optionally, the material for the housing can be selected from a group consisting of Aluminium, steel, Iron, Nickel, Copper, Zinc, Tin, or any combination thereof. In accordance to a specific embodiment of the disclosure, the housing is made of Aluminium. The housing may be painted or may have a protective coating such as alloy coating. In accordance with an embodiment, the process of anodizing may be used to coat the housing such as to form a protective coating of Aluminum Oxide on the surface of the housing. The housing may be of any suitable size that can be conveniently attached to the mount of the tool interface assembly without affecting ease of the surgical operation. The housing may be of a suitable thickness providing sufficient strength. The housing may be of any suitable shape (suitably similar to the mount of the tool interface assembly) such that the ease of affixing the aforesaid is maintained. In accordance with an embodiment of the disclosure, the housing is substantially of a square shape where a bottom end of the housing is substantially larger than a top end of the housing and the body of the housing is substantially tapered towards the top end of the housing.

In an embodiment, the locking plate may be a flat plate containing at least one aperture configured to receive and engage with portions received by the aperture of the housing. According to a specific embodiment, a pair of apertures are provided in the locking plate.

The pair of apertures may be configured to receive the holding portion of the cannula sterile adaptor having the attachment portions of the cannula contained therein. According to a specific embodiment, a pair of aperture are provided to engage with the locking prongs of the attachment portions of the cannula. The disclosure may function with one aperture and one holding portion also. According to an embodiment, the shape of the apertures corresponds to the shape of the holding portions of the cannula sterile adaptor and the attachment portions of the cannula, such as to facilitate mounting of the cannula in the housing of the cannula securing assembly. According to an embodiment, the diameter of the apertures of the locking plate corresponds with the diameter of the apertures. The locking plate is capable of translation motion within the housing.

In another embodiment, the locking plate may be made of any suitable resilient material such as a metal or an alloy. Optionally, the material for the locking plate can be selected from a group consisting of Aluminium, steel, Iron, Nickel, Copper, Zinc, Tin, or any combination thereof. In accordance to a specific embodiment of the disclosure, the locking plate is made of Aluminium. The locking plate may be painted or may have a protective coating such as alloy coating. In accordance with an embodiment, the process of anodizing may be used to coat the locking plate such as to form a protective coating of Aluminum Oxide on the surface of the locking plate. The locking plate may be of any suitable size that can be conveniently attached to the housing without affecting ease of the surgical operation. The locking plate may be of a suitable thickness providing sufficient strength.

In yet another embodiment, the locking plate also may comprise of a releasing means at one end of the locking plate where the releasing mechanism (namely, "*releasing means*") is configured to be exposed, namely in exposed when in operation, on an exterior surface of the housing. According to an embodiment, the releasing mechanism is shaped like a button and when pressed or applied pressure can be configured to disengage the cannula with the cannula securing assembly. According to a specific embodiment, the releasing mechanism is a shutter-release button.

In an embodiment, the retainer plate may be affixed to an interior surface of the housing such that the retainer plate is fixed over the locking plate for facilitating a reciprocating movement of the locking plate. The retainer plate fixes when in operation, namely is configured to fix, the locking plate in one position such that the locking plate is secured within the housing. The retainer plate may be bolted to the housing. Alternatively, the retainer plate may be riveted, screwed or a combination thereof to the housing. Furthermore, slots are provided on an internal surface within the housing for the retainer plate to be bolted therein.

In another embodiment, the retainer plate may be made of any suitable resilient material such as a metal or an alloy. Optionally, the material for the retainer plate can be selected from a group consisting of Aluminium, steel, Iron, Nickel, Copper, Zinc, Tin, or any combination thereof. In accordance to a specific embodiment of the disclosure, the retainer plate is made of Aluminium. The retainer plate may be painted or may have a protective coating such as alloy coating. In accordance with an embodiment, the process of anodizing may be used to coat the retainer plate such as to form a protective coating of Aluminum Oxide on the surface of the retainer plate. The retainer plate may be of any suitable size that can be conveniently attached to the housing without affecting ease of the surgical operation. The retainer plate may be of a suitable thickness providing sufficient strength.

In an embodiment, the cannula securing housing may comprise of a back cover that close, when in operation, the housing from one end. According to an embodiment, the back cover may be a flat plate. The back cover may be bolted to the housing. Alternatively, the back cover may be riveted, screwed or a combination thereof to the housing. In a specific embodiment, the slots are provided on an internal surface within the housing for the back cover to be bolted therein.

In an embodiment, the back cover may also comprise a protruding slot -on an internal surface of the back cover such that when the housing is closed with the back cover, the protruding slot remains within the housing.

In another embodiment, the back cover may be made of any suitable resilient material such as a metal or an alloy. Optionally, the material for the back cover can be selected from a group consisting of Aluminium, steel, Iron, Nickel, Copper, Zinc, Tin, or any combination thereof. In accordance to a specific embodiment of the disclosure, the back cover is made of Aluminium. The back cover may be painted or may have a protective coating such as alloy coating. The back cover may be of any suitable size that can be conveniently attached to the housing without affecting ease of the surgical operation. The back cover may be of a suitable thickness providing sufficient strength.

In an embodiment, the compression mechanism, namely "*compression means*"*,* may be provided for biasing the locking plate to facilitate engaging of the cannula with the housing of the cannula securing assembly. According to an embodiment, the compression mechanism is a flat spring.

In another embodiment, the compression mechanism at a first end may be bolted to the protruding slot of the back cover such that the compression mechanism is fixed within the housing. Alternatively, the compression mechanism may be riveted, screwed or a combination thereof to the protruding slot of the back cover. The compression mechanism at a second end engages with the locking plate such that the compression mechanism exerts a tension on the locking plate, wherein the locking plate stays in a locked position. When the locking plate is in the locked position, the cannula is engaged within the pair of aperture of the locking plate.

Herein, biasing the locking plate may be referred as the spring applying a biasing force on the locking plate at all times for the locking plate to engage the cannula within the housing of the cannula securing assembly. Furthermore, the spring may apply the biasing force on the locking plate such that the locking prongs of the attachment portions of the cannula are constantly engaged within the pair of aperture of the locking plate.

On the other hand, when the releasing mechanism is pressed, the tension applied by the compression mechanism is disengaged or interrupted which facilitates the disengagement of cannula from the pair of aperture of the locking plate. Herein, the biasing force may be depressed or released such that the locking prongs of the attachment portions of the cannula may be disengaged from the pair of aperture of the locking plate and the cannula pops out as a result of the disengagement. This stage may be referred as when the locking plate is in an unlocked position.

Furthermore, one or more sensors may be positioned towards a top end of the housing and may when in operation, namely may be configured to, measure force exerted on the cannula and to detect a presence of the cannula within the housing. According to a specific embodiment, force sensors may be used. The one or more sensors may be positioned on a PCB (printed circuit board) which may be bolted to an internal surface within the housing. Alternatively, the PCB may be riveted, screwed or a combination thereof to the housing. The slots are provided on an internal surface within the housing for the PCB to be bolted therein.

In an embodiment, the PCB may be fixed to the locking plate with the help of a connector. Furthermore, force sensor is connected on the PCB to measure the force applied on the cannula.

According to an exemplary embodiment, the one or more sensors may measure the force exerted by the surgeon on the cannula and also on the tissues of the patient's body wall around the cannula during a surgery. Furthermore, during the surgery, the force exerted by the surgeon on the cannula is transferred to the one or more sensors via a shaft connected at a proximal end of the cannula. The shaft presses the one or more sensors and the force is detected by the one or more sensors and one or more sensor signals indicative of applied force may be conveyed to the display of the vision cart. Furthermore, the one or more signals may also be displayed at the viewing apparatus at the surgeon console. Furthermore, a threshold may be set and whenever the force exerted on the cannula increases above the threshold, an alert by way of an alarm or a message is displayed to the surgeon or assistants near the surgeon. By way of example, the one or more sensors may also include a load cell sensor, which measures the deformation of the tissues of the patient's body wall around the cannula into an electrical signal and send an alert to the surgeon if the deformation is above a threshold. According to a specific embodiment, the attachment portions are the portion of cannula that is received by the housing.

The surgical system disclosed herein comprises of a cannula and a cannula securing assembly. The cannula securing assembly comprises of a housing including at least one aperture to receive a portion of the cannula and a locking plate including at least one aperture configured to engage with the portion of cannula received in the at least one aperture of the housing. The locking plate further includes a releasing mechanism, namely "releasing means", at one end of the locking plate wherein the releasing mechanism is exposed when in operation, namely is configured to be exposed, on an exterior surface of the housing. The cannula securing assembly further comprises of a compression mechanism, namely "compression means", affixed to a slot on an interior surface of the housing and engages when in operation, namely is configured to engage, with the locking plate, wherein the compression mechanism biases, namely is configured for biasing, the locking plate to facilitate engaging of the cannula with the cannula securing assembly and the releasing mechanism disconnects when in operation, namely is configured for disconnecting, the biasing of the locking plate to facilitate disengaging of the cannula with the cannula securing assembly.

### DETAILED DESCRIPTION OF THE DRAWINGS

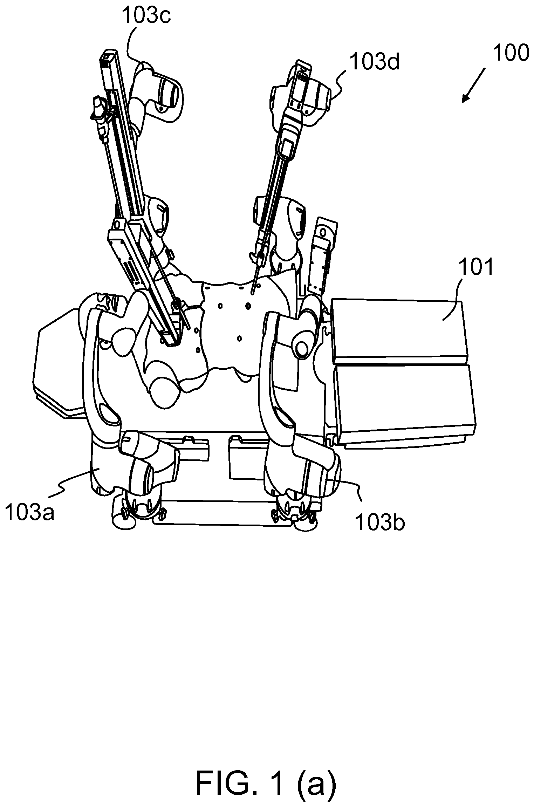

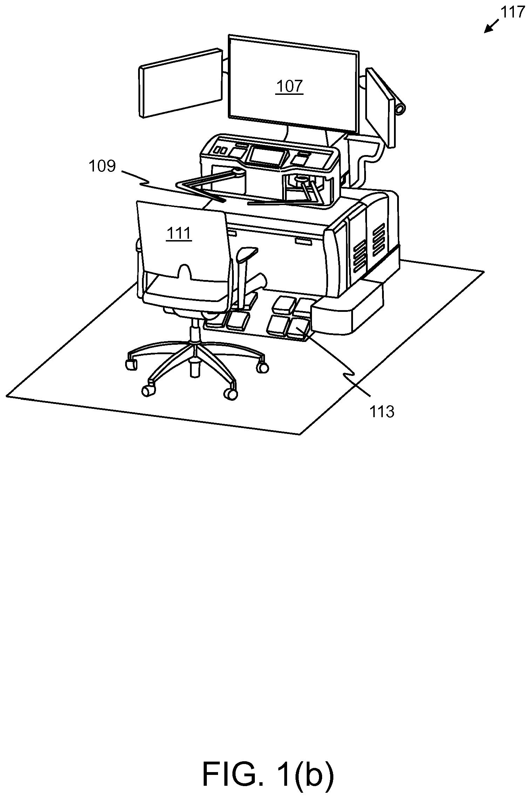

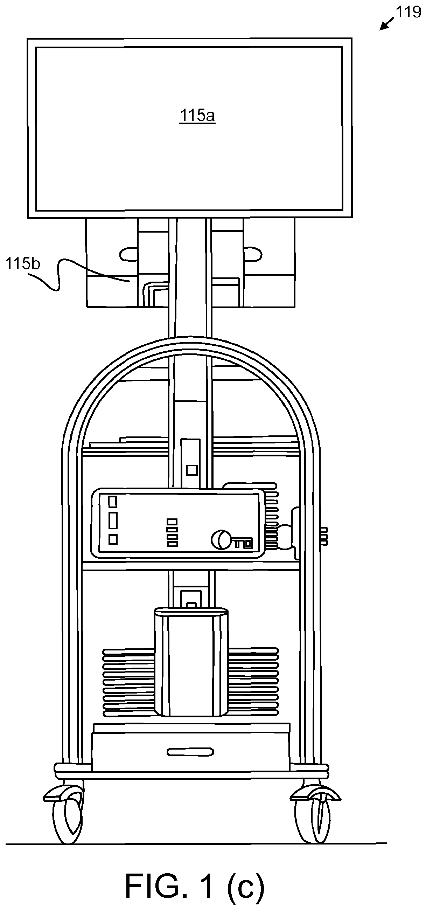

In FIG. 1(a), there is provided an illustration of a schematic diagram of multiple robotic arms of a robotic surgical system in accordance with an embodiment of the present disclosure. Specifically, FIG. 1 provides an illustration of the robotic surgical system **100** that may have four robotic arms **103a, 103b, 103c, 103d** mounted around a patient cart **101.** The four-robotic arms **103a, 103b, 103c, 103d** as depicted in FIG. 1 are for illustration purpose and the number of robotics arms may vary depending upon the type of surgery or the robotic surgical system. The four robotic arms **103a, 103b, 103c, 103d** may be mounted along the patient cart **101** and may be arranged in a different manner, but not limited to the robotic arms **103a, 103b, 103c, 103d** mounted on the patient cart **101** or the robotic arms **103a, 103b, 103c, 103d** separately mounted on a movable mechanism (namely "*movable means*") or the robotic arms **103a, 103b, 103c, 103d** mechanically and/ or operationally connected with each other or the robotic arms **103a, 103b, 103c, 103d** connected to a central body such that the robotic arms **103a, 103b, 103c, 103d** branch out of the central body (not shown).

In FIG. 1(b), there is provided an illustration of a schematic diagram of a surgeon console of the robotic surgical system in accordance with an embodiment of the present disclosure. The surgeon console **117** may aid the surgeon to operate remotely the patient lying on the patient cart **101** (such as the patient cart in FIG.1(a)) by controlling the robotic arms **103a, 103b, 103c, 103d** (such as the robotic arms in FIG.1(a)) around the body of the patient. The surgeon console **117** may control when in operation, namely be configured to control, the movement of surgical instruments (not shown) while the instruments are inside the patient body. The surgeon console **117** may comprise at least an adjustable viewing apparatus **107** but not limited to 2D/ 3D monitors, wearable viewing apparatus (not shown) and in combination thereof. The surgeon console **117** may be equipped with multiple displays which would not only show 3D high definition (HD) endoscopic view of a surgical site at the patient cart **101,** but may also shows additional information from various medical equipment's which surgeon may use during the robotic surgery. Furthermore, the viewing apparatus **107,** namely "viewing means", may provide various modes of the robotic surgical system **100,** but not limited to identification and number of robotic arms attached, current surgical instrument type attached, current instrument tool tip position, collision information along with medical data like ECG, ultrasound display, fluoroscopic images, CT, MRI information. The surgeon console **117** may further comprise a mechanism for controlling the robotics arms, but not limited to one or more hand controllers **109,** one or more-foot controllers **113,** a clutch mechanism (not shown), and any combination thereof. The hand controllers **109** at the surgeon console **117** are required to capture seamlessly and transfer complex actions performed by surgeon giving the perception that the surgeon is directly articulating the surgical tools. The different controllers may be required for different purposes during the surgery. In some embodiments, the hand controllers **109** may be one or more manually-operated input devices, such as a joystick, exoskeletal glove, a powered and gravity-compensated manipulator, or the like. These hand controllers **109** control teleoperated motors which, in turn, control the movement of the surgical instruments attached to the robotic arms. The surgeon may sit on a resting apparatus such as a chair **111,** as depicted in FIG. 1(b), while controlling the surgeon console **117.** The chair **111** may be adjustable in height, elbow rest, and the like according to the ease of the surgeon and also various control means may be provided on the chair **111.** Furthermore, the surgeon console **117** may be at a single location inside an operation theatre or may be distributed at any other location (for example, remotely) in the hospital provided connectivity to the robotics arms is maintained.

In FIG. 1(c), there is provided an illustration of a schematic diagram of a vision cart of the robotic surgical system in accordance with an embodiment of the present disclosure. The vision cart **119** may be configured to display the 2D and/or 3D view of the operation captured by an endoscope. The vision cart **119** may be adjusted at various angles and heights depending upon the ease of view. The vison cart **119** may have various functionalities, but not limited to providing touch screen display, preview/recording/playback provisions, various inputs/ outputs means, 2D to 3D converters, and the like. The vision cart **119** may include a vision system or displays **115a, 115b** that enables a spectator or other non-operating surgeons/assistants to view a surgical site from outside the patient's body. One of the robotics arms typically engage a camera that has a video-image-capture function (i.e., an endoscopic camera) for displaying the captured images on the vision cart **119.** In some robotic surgical system configurations, the camera includes optics that transfer the images from the distal end of the camera to one or more imaging sensors (e.g., CCD or CMOS sensors) outside of the patient's body. Alternatively, one or more imaging sensors employed may be positioned at the distal end of the camera, and the signals produced by the one or more sensors may be transmitted along a wire or wirelessly for processing and display on the vision cart **119.**

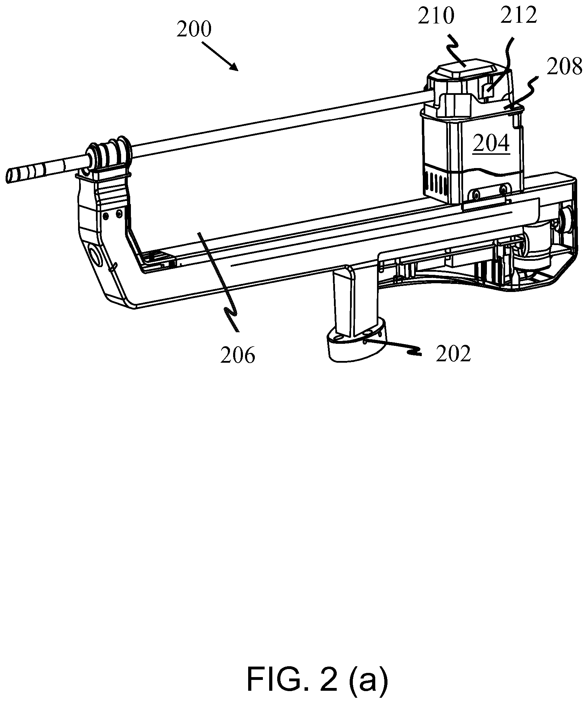

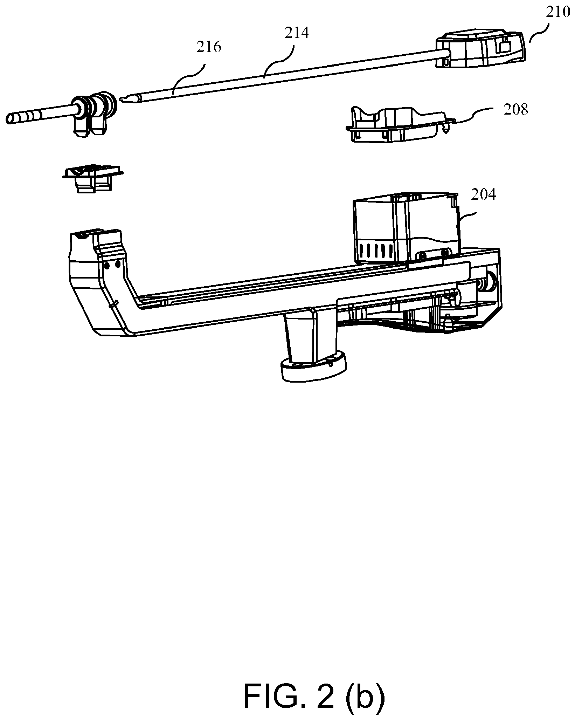

In FIG. 2(a), there is provided an illustration of a perspective view of the tool interface assembly and FIG. 2(b), wherein there is illustrated an exploded view of the tool interface assembly in accordance with an embodiment of the present disclosure. The tool interface assembly **200** may be mounted on any of the robotic arm of a robotic surgical system (such as the robotic surgical system **100** of FIG. 1). The tool interface assembly **200** may be the main component for performing the robotic surgery on a patient.

The tool interface assembly **200,** as depicted by any of the FIG. 2(a) or FIG. 2(b), may comprise of an Arm and Tool Interface (ATI) connector **202** which facilitates the tool interface assembly **200** to connect operationally with the robotic arm (shown in FIG. 1(a)). The tool interface assembly **200** may further comprise an actuator assembly **204** mounted on a guiding mechanism and capable of linearly moving along the guiding mechanism. The guiding mechanism depicted in FIGs. 2(a) and 2(b) is a guide rail **206.** The movement of the actuator assembly **204** along the guide rail **206** is controlled by the surgeon with the help of hand controllers on the surgeon console **117** as shown in FIG. 1(b). A sterile adapter assembly **208** is releasably mounted on the actuator assembly **204** to separate a non-sterile part of the robotic arm from a sterile surgical tool assembly **210.** A locking mechanism (not shown) may be provided to releasably lock and unlock the sterile adapter assembly **208** with the actuator assembly **204.** The sterile adaptor assembly **208** may detachably engage with the actuator assembly **204** which drives and controls the sterile surgical instrument in a sterile field. In another embodiment, the sterile surgical tool assembly **210** also may releasably lock/ unlock or engage/disengage with the sterile adapter assembly **208** via use of, namely "*by means of*", a push button **212.**

The sterile surgical tool assembly **210** may include a shaft **214** and end effectors **216.** The end effector **216** may comprises a surgical instrument integrated with the end effector **216** or the end effector **216** may inclues, namely be configured to include, an attachment mechanism, namely "attachment means", to attach a surgical instrument (not shown) where the end effector **216** may act as a pluggable device. The surgical instrument may be associated with one or more surgical tasks, such as a forcep, a needle driver, a shears, a bipolar cauterizer, a tissue stabilizer or retractor, a clip applier, an anastomosis device, an imaging device (e.g., an endoscope or ultrasound probe), and the like. Some surgical instruments further provide an articulated support (sometimes referred to as a "wrist") for the sterile surgical tool assembly **210,** such that the position and orientation of the surgical tool assembly **210** may be manipulated with one or more mechanical degrees of freedom in relation to the instrument's shaft **214.** Furthermore, the end effectors **216** may include a functional mechanical degree of freedom, such as jaws that open or close, or a knife that translates along a path. The sterile surgical tool assembly **210** may also contain stored (e.g., on a memory inside the instrument) information that may be permanent or may be updatable by the robotic surgical system **100.**

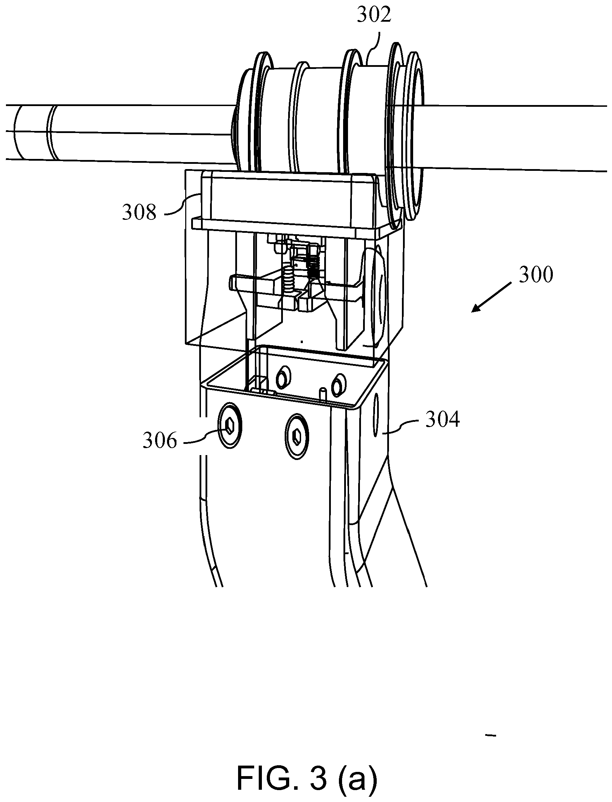

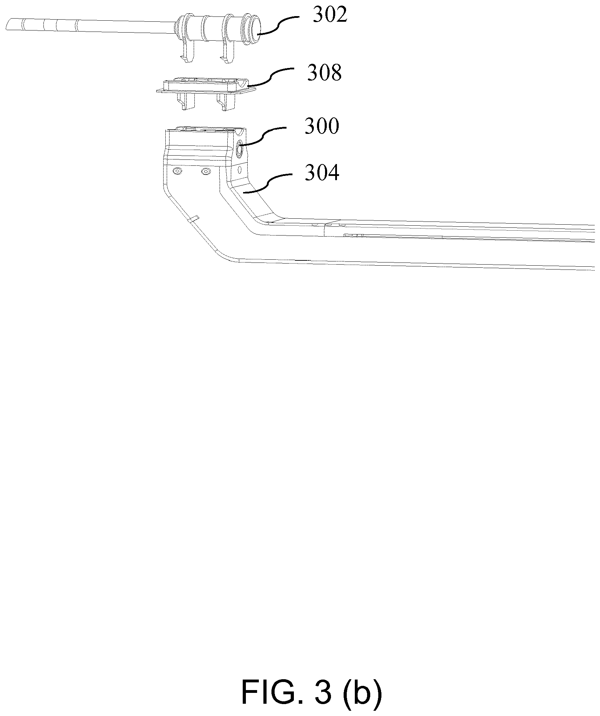

In FIG. 3(a) and 3(b), there are provided illustrations of a perspective view and an exploded view of a cannula securing assembly attached to a cannula and a cannula sterile adaptor in accordance with an embodiment of the present disclosure. The cannula securing assembly **300** may be affixed to the body of a tool interface assembly (such as the tool interface assembly **200** of FIG. 2) and may grip of secure when in operation, namely may be configured to grip or secure, the cannula **302** such that cannula **302** is stable while performing surgical operations. The cannula securing assembly **300** may be affixed to a mount 304 of the tool interface assembly 200 by bolts 306. Alternatively, the cannula securing assembly 300 may be riveted, screwed or a combination thereof to the mount 304 of the tool interface assembly 200. According to a specific embodiment of the present disclosure, mutually opposite sides of the cannula securing assembly 300 may be bolted on respective opposite sides of the mount 304 of the tool interface assembly 200. Furthermore, in FIG. 3(b), there is provided an illustration of an exploded view of the cannula securing assembly 300, the cannula 302 and the cannula sterile adaptor 308 in distributed manner. A detailed explanation of the assembly is provided in the following description and accompanying diagrams.

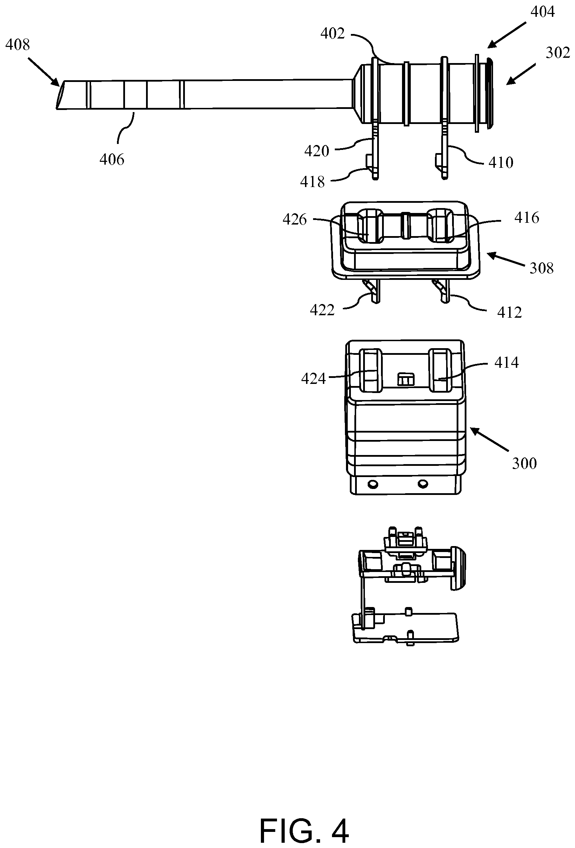

In FIG. 4, there is provided an illustration of an exploded view of the cannula, the cannula sterile adaptor, and the cannula securing assembly in accordance with an embodiment of the present disclosure. The cannula 302 may comprise of a hollow body. The cannula **302** is fixed to a shaft (such as the shaft **214** of FIG. 2) at a desired angle and precludes shifting, twisting or any axial movement of the shaft **214** once received by the cannula **302.** The cannula **302** may be inserted through an opening in a patient's body to a surgical site.

The cannula **302** may include a bowl section **402** forming a proximal end **404** of cannula **302,** and a tube **406** may extend from the bowl section **402** to a distal end **408** of cannula **302.** The proximal and distal directions with respect to the orientation of FIG. 4 are labeled. According to a specific embodiment as illustrated in FIG. 4, the tube **406** may have a length L, the distal end **408** and proximal end **404** may have a diameter D, Di, and the bowl section **402** having a circumference C, each of which may vary depending upon a desired application of the cannula **302.** Furthermore, as shown in the specific embodiment of FIG. 4, the tube **406** may be straight, although the exemplary cannula embodiments described herein are not limited to a straight tube. For example, alternatively, the tube **406** may be a curved tube having a substantially curved longitudinal axis. The cannula **302** may also comprise an attachment portion **410** which encloses the bowl section **402** and may have a protruding end. The protruding end of the attachment portion **410** may be provided with locking prongs **418** which help the attachment portion **410** in engaging or disengaging from the cannula securing assembly **300.**

The cannula sterile adaptor **308** may be connected to the cannula securing assembly **300,** such as by inserting holding portions **412, 422** of the cannula sterile adaptor **308** into respective apertures **414, 424** of the cannula securing assembly **300.** According to a specific embodiment as illustrated in FIG. 4, a pair of holding portions **412, 422** and a pair of respective recesses **414, 424** are provided. The disclosure may function with one holding portion and one recess also. According to an embodiment, the shape of the holding portion **412, 422** corresponds to the shape of the recess **414, 424** of the cannula securing assembly **300** such as to facilitate mounting of the cannula sterile adaptor **308** in the cannula securing assembly **300.**

The cannula sterile adaptor **308** may facilitate forming a boundary between a sterile region and a non-sterile region, which is discussed earlier. For example, a surgical drape (not shown) may be attached to the cannula sterile adaptor **308** to separate a sterile side from a non-sterile side of the drape.

The attachment portions **410, 420** of the cannula **302** may be structured to fit inside openings **416, 426** of the cannula sterile adaptor **308,** so that the cannula **302** remains on sterile side of the drape. Furthermore, when the cannula sterile adaptor **308** has been connected to the cannula securing assembly **300,** and attachment portions **410, 420** are inserted into the openings **416, 426** of the cannula sterile adaptor **308,** the cannula **302** may also be connected to the cannula securing assembly **300** so that the cannula **302** may be held by the cannula securing assembly **300** during a surgical procedure. The disclosure may function with one attachment portion and one opening also.

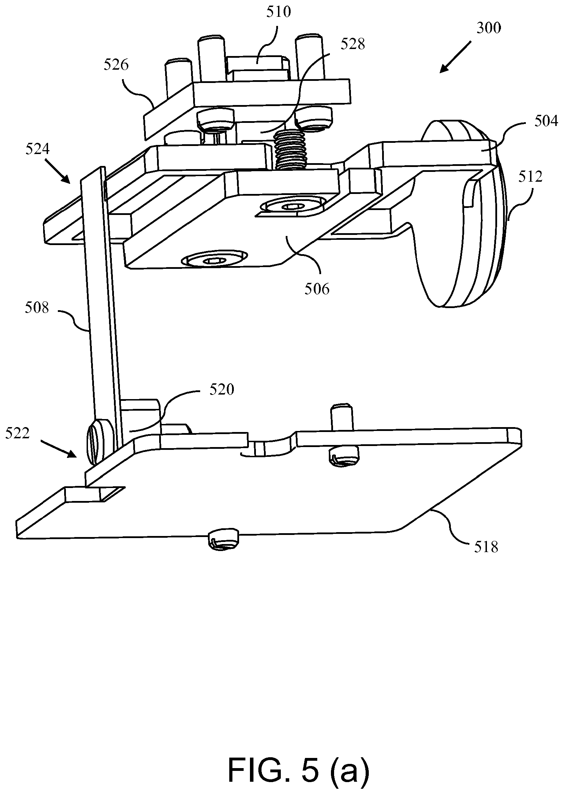

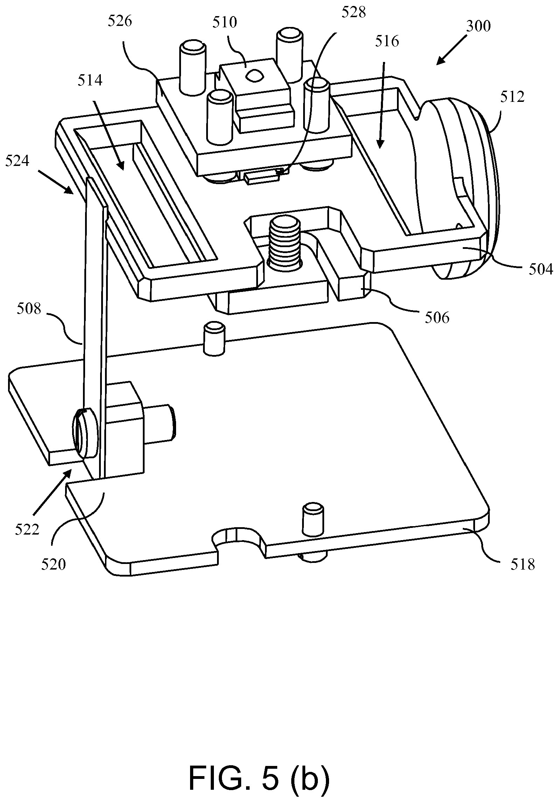

In FIG. 5(a) and 5(b), there are provided illustration of components of the cannula securing assembly in accordance with an embodiment of the present disclosure. A cannula securing housing (such as the cannula securing housing **300** of FIG. 3) comprising of a housing **600,** a locking plate **504,** a retaining plate **506,** a compression mechanism (namely, *"compression means")* **508,** and one or more sensors **510.** Furthermore, the locking plate **504** may be a flat plate containing at least one aperture **514** configured to receive and engage with portions received by the aperture **602** of the housing **600.** According to a specific embodiment as illustrated in FIG. 5(a) and 5(b), a pair of aperture **514, 516** are provided in the locking plate **504.**

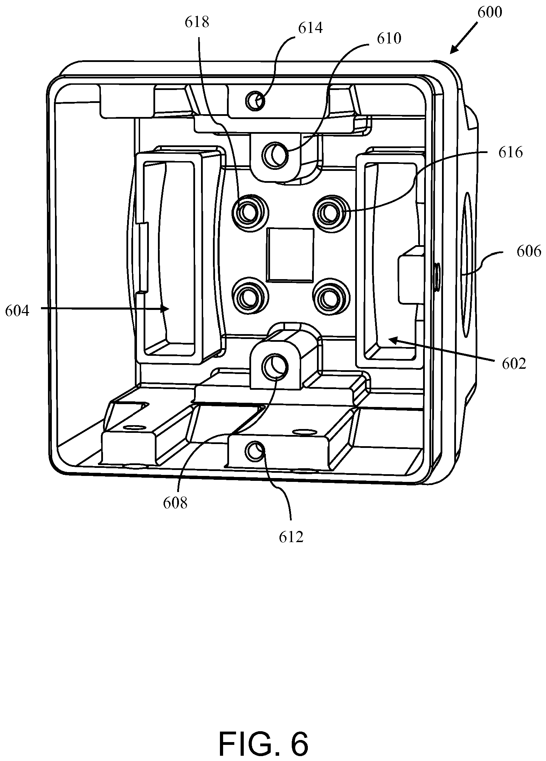

Referring now to FIG. 6, the housing **600** may be a shell for enclosing the parts of the cannula securing assembly (such as the cannula securing assembly **300** of FIG. 3). The housing **600** also includes an aperture **602** (such as the apertures **414** and **424** of FIG. 4) to receive a holding portion (such as the holding portion **412** of FIG. 4) of a cannula sterile adaptor (such as the cannula sterile adaptor **308** of FIG. 3) having a the attachment portion (such as the attachment portion **410** of FIG. 4) of a cannula (such as the cannula **302** of FIG. 3) contained therein. According to a specific embodiment as illustrated in FIG. 6, a pair of apertures **602** to **604** is provided to receive a pair of holding portions **412.** The cannula securing assembly **300** may function with one recess and one holding portion **412** also. According to an embodiment, the shape of the aperture **602** to **604** corresponds to the shape of the holding portion **412** of the cannula sterile adaptor **308** such as to facilitate mounting of the cannula **302** in the housing **600** of the cannula securing assembly **300.** Furthermore, in FIG. 6, the housing **600** comprises an opening **606** for receiving a releasing mechanism (namely, *"releasing means*") (such as the releasing mechanism **512** of FIG. 5) of a locking plate (such as the locking plate **504** of FIG.4). According to an embodiment, the opening **606** is circular in shape and the diameter of the opening **606** is substantially larger than the diameter of the releasing mechanism **512** of the locking plate **504** such that the releasing mechanism **512** fits well within the opening **606.** Furthermore, as illustrated in FIG. 6, slots **608** to **618** are provided on an internal surface within the housing **600** for a retainer plate (such as the retainer plate **506** of FIG. 5) to be bolted therein.

The foregoing descriptions of exemplary embodiments of the present disclosure have been presented for purposes of illustration and description. They are not intended to be exhaustive or to limit the disclosure to the precise forms disclosed, and obviously many modifications and variations are possible in light of the above teaching. The exemplary embodiment was chosen and described in order to best explain the principles of the disclosure and its practical application, to thereby enable others skilled in the art to best utilize the disclosure and various embodiments with various modifications as are suited to the particular use contemplated. It is understood that various omissions, substitutions of equivalents are contemplated as circumstance may suggest or render expedient but is intended to cover the application or implementation without departing from the scope of the claims of the present disclosure.

Benefits, other advantages, and solutions to problems have been described above with regard to specific embodiments. However, the benefits, advantages, solutions to problems, and any component(s) that may cause any benefit, advantage, or solution to occur or become more pronounced are not to be construed as a critical, required, or essential feature or component of any or all the claims.

While specific language has been used to describe the disclosure, any limitations arising on account of the same are not intended. As would be apparent to a person in the art, various working modifications may be made to the apparatus in order to implement the inventive concept as taught herein.

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Reference to the singular is also to be construed to relate to the plural.

## Claims

1. A cannula securing assembly (300) for selectively engaging and disengaging a cannula (302) in a surgical system (100), the cannula securing assembly (300) comprising:
- a housing (600) including at least one aperture (602) to receive a portion (410) of the cannula (302);
- a locking plate (504) affixed within the housing (600), including at least one aperture (516) that engages when in operation with the portion (410) of cannula (302) received in the at least one aperture (602) of the housing (600) and a releasing mechanism (512) at one end of the locking plate (504), wherein the releasing mechanism (512) is exposed when in operation on an exterior surface of the housing (600); and **characterized in that** the cannula securing assembly further comprises
- a compression mechanism (508) affixed to a slot (520) on an interior surface of the housing (600) at a first end and engaging the locking plate (504) at a second end ,
wherein the compression mechanism (508) biases the locking plate (504) to facilitate engaging of the cannula (302) with the cannula securing assembly (300) and the releasing mechanism (512) disconnects when in operation the biasing of the locking plate (504) to facilitate disengaging of the cannula (302) with the cannula securing assembly (300).

2. A cannula securing assembly (300) of claim 1, further comprising one or more sensors (510) to measure force exerted on the cannula (302) and detect a presence of the cannula (302) in the cannula securing assembly (300).

3. A cannula securing assembly (300) of claim 2, wherein the one or more sensors (510) are positioned on a Printed Circuit Board (PCB) (526), wherein the PCB (526) is affixed to an internal surface of the housing (600).

4. A cannula securing assembly (300) of claim 1, further comprising a retainer plate (506) affixed to an interior surface of the housing (600), such that the retainer plate (506) is fixed over the locking plate (504) for facilitating a reciprocating movement of the locking plate (504).

5. A cannula securing assembly (300) of claim 1, wherein the compression mechanism (508) is a flat spring.

6. A cannula securing assembly (300) of claim 1, wherein the cannula securing assembly (300) is made of Aluminium.

7. A cannula securing assembly (300) of claim 1, wherein the cannula securing assembly (300) receives when in operation a cannula sterile adaptor (308) such that the cannula sterile adaptor (308) is positioned between the cannula securing assembly (300) and cannula (302).

8. A cannula securing assembly (300) of claim 1, wherein the cannula sterile adaptor (308) is connected to a drape such that the cannula sterile adaptor (308) and the drape forms a boundary between a sterile region and a non-sterile region.

9. A cannula securing assembly (300) of claim 1, wherein the cannula (302) comprises a bowl section (402) forming a proximal end (404) of the cannula (302) and a tube (406) extending from the bowl section (402) to a distal end (408) of the cannula (302).

10. A cannula securing assembly (300) of claim 1, wherein the cannula (302) further comprises an attachment portion (410), (420) having locking prongs (418) configured to engage or disengage the cannula (302) from the cannula securing assembly (300).

11. A cannula securing assembly (300) of claim 1, wherein the locking plate (504) is a flat plate.

## Patentansprüche

1. Kanülenbefestigungsbaugruppe (300) zum selektiven Einsetzen und Lösen einer Kanüle (302) in einem chirurgischen System (100), **dadurch gekennzeichnet, dass** die Kanülenbefestigungsbaugruppe (300) Folgendes umfasst:
- ein Gehäuse (600), das mindestens eine Aussparung (602) einschließt, um einen Abschnitt (410) der Kanüle (302) zu empfangen;
- eine innerhalb des Gehäuses (600) befestigte Verriegelungsplatte (504), die mindestens eine Aussparung (516), die im Betrieb mit dem Abschnitt (410) der Kanüle (302), der in der mindestens einen Öffnung (602) des Gehäuses (600) aufgenommen ist, in Eingriff kommt, und einen Freigabemechanismus (512) an einem Ende der Verriegelungsplatte (504) einschließt, wobei der Freigabemechanismus (512) im Betrieb an einer äußeren Oberfläche des Gehäuses (600) freiliegt; und
- einen Kompressionsmechanismus (508), der an einem Schlitz (520) auf einer inneren Oberfläche des Gehäuses (600) an einem ersten Ende und an der Verriegelungsplatte (504) an einem zweiten Ende befestigt ist,
wobei der Kompressionsmechanismus (508) die Verriegelungsplatte (504) vorspannt, um das Ineinandergreifen der Kanüle (302) mit der Kanülenbefestigungsbaugruppe (300) zu erleichtern, und der Freigabemechanismus (512) im Betrieb die Vorspannung der Verriegelungsplatte (504) aufhebt, um das Lösen der Kanüle (302) von der Kanülenbefestigungsbaugruppe (300) zu erleichtern.

2. Kanülenbefestigungsbaugruppe (300) nach Anspruch 1, die ferner einen oder mehrere Sensoren (510) umfasst, um die auf die Kanüle (302) ausgeübte Kraft zu messen und eine Anwesenheit der Kanüle (302) in der Kanülenbefestigungsbaugruppe (300) festzustellen.

3. Kanülenbefestigungsbaugruppe (300) nach Anspruch 2, wobei der eine oder die mehreren Sensoren (510) auf einer Leiterplatte (PCB) (526) positioniert sind, wobei die PCB (526) an einer inneren Oberfläche des Gehäuses (600) befestigt ist.

4. Kanülenbefestigungsbaugruppe (300) nach Anspruch 1, die ferner eine Halterungsplatte (506) umfasst, die an einer inneren Oberfläche des Gehäuses (600) befestigt ist, sodass die Halterungsplatte (506) über der Verriegelungsplatte (504) befestigt ist, um eine Hin- und Herbewegung der Verriegelungsplatte (504) zu ermöglichen.

5. Kanülenbefestigungsbaugruppe (300) nach Anspruch 1, wobei der Kompressionsmechanismus (508) eine Flachfeder ist.

6. Kanülenbefestigungsbaugruppe (300) nach Anspruch 1, wobei die Kanülenbefestigungsbaugruppe (300) aus Aluminium hergestellt ist.

7. Kanülenbefestigungsbaugruppe (300) nach Anspruch 1, wobei die Kanülenbefestigungsbaugruppe (300) im Betrieb einen sterilen Kanülenadapter (308) empfängt, sodass der sterile Kanülenadapter (308) zwischen der Kanülenbefestigungsbaugruppe (300) und der Kanüle (302) positioniert ist.

8. Kanülenbefestigungsbaugruppe (300) nach Anspruch 1, wobei der sterile Kanülenadapter (308) mit einem Tuch verbunden ist, sodass der sterile Kanülenadapter (308) und das Tuch eine Grenze zwischen einer sterilen Region und einer unsterilen Region bilden.

9. Kanülenbefestigungsbaugruppe (300) nach Anspruch 1, wobei die Kanüle (302) einen schalenförmigen Abschnitt (402), der ein proximales Ende (404) der Kanüle (302) bildet, und ein Rohr (406) umfasst, das sich von dem schalenförmigen Abschnitt (402) zu einem distalen Ende (408) der Kanüle (302) erstreckt.

10. Kanülenbefestigungsbaugruppe (300) nach Anspruch 1, wobei die Kanüle (302) ferner einen Abschnitt (410), (420) mit Verriegelungszinken (418) aufweist, die so konfiguriert sind, dass sie die Kanüle (302) mit der Kanülenbefestigungsbaugruppe (300) in Eingriff bringen oder von ihr lösen.

11. Kanülenbefestigungsbaugruppe (300) nach Anspruch 1, wobei die Verriegelungsplatte (504) eine flache Platte ist.

## Revendications

1. Ensemble de sécurisation d'une canule (300) pour engager sélectivement et désengager une canule (302) dans un système chirurgical (100), **caractérisé en ce que** l'ensemble de sécurisation d'une canule (300) comprenant :
- un boîtier (600) comprenant au moins une ouverture (602) pour recevoir une partie (410) de la canule (302) ;
- une plaque de verrouillage (504) fixée à l'intérieur du boîtier (600), comprenant au moins une ouverture (516) qui s'engage lors du fonctionnement avec la partie (410) de la canule (302) reçue dans au moins une ouverture (602) du boîtier (600) et un mécanisme de libération (512) à une extrémité de la plaque de verrouillage (504), le mécanisme de libération (512) étant exposé lorsqu'il fonctionne sur une surface extérieure du boîtier (600) ; et
- un mécanisme de compression (508) fixé à une fente (520) sur une surface intérieure du boîtier (600) à une première extrémité et fixé à la plaque de verrouillage (504) à une seconde extrémité,
dans lequel le mécanisme de compression (508) sollicite la plaque de verrouillage (504) pour faciliter l'engagement de la canule (302) avec l'ensemble de sécurisation d'une canule (300) et le mécanisme de libération (512) se déconnecte lors du fonctionnement de la sollicitation de la plaque de verrouillage (504) ) pour faciliter le désengagement de la canule (302) avec l'ensemble de sécurisation d'une canule (300).

2. Ensemble de sécurisation d'une canule (300) selon la revendication 1, comprenant en outre un ou plusieurs capteurs (510) pour mesurer la force exercée sur la canule (302) et détecter une présence de la canule (302) dans l'ensemble de sécurisation d'une canule (300).

3. Ensemble de sécurisation d'une canule (300) selon la revendication 2, dans lequel le ou les capteurs (510) sont positionnés sur une Carte de Circuit Imprimé (PCB) (526), dans lequel la PCB (526) est fixée à une surface interne du boîtier ( 600).

4. Ensemble de sécurisation d'une canule (300) selon la revendication 1, comprenant en outre une plaque de retenue (506) fixée à une surface intérieure du boîtier (600), de telle sorte que la plaque de retenue (506) soit fixée sur la plaque de verrouillage (504) pour faciliter un mouvement réciproque de la plaque de verrouillage (504).

5. Ensemble de sécurisation d'une canule (300) selon la revendication 1, dans lequel le mécanisme de compression (508) est un ressort plat.

6. Ensemble de sécurisation d'une canule (300) selon la revendication 1, dans lequel l'ensemble de sécurisation d'une canule (300) est fabriqué en Aluminium.

7. Ensemble de sécurisation d'une canule (300) selon la revendication 1, dans lequel l'ensemble de sécurisation d'une canule (300) reçoit lorsqu'il est en fonctionnement un adaptateur stérile pour canule (308) de telle sorte que l'adaptateur stérile pour canule (308) est positionné entre l'ensemble de sécurisation d'une canule (300) et la canule (302).

8. Ensemble de sécurisation d'une canule (300) selon la revendication 1, dans lequel l'adaptateur stérile pour canule (308) est connecté à un champ de telle sorte que l'adaptateur stérile pour canule (308) et le champ forment une limite entre une région stérile et une région non stérile.

9. Ensemble de sécurisation d'une canule (300) selon la revendication 1, dans lequel la canule (302) comprend une section de bol (402) formant une extrémité proximale (404) de la canule (302) et un tube (406) s'étendant depuis la section de bol (402) à une extrémité distale (408) de la canule (302).

10. Ensemble de sécurisation d'une canule (300) selon la revendication 1, dans lequel la canule (302) comprend en outre une partie de fixation (410), (420) ayant des broches de verrouillage (418) configurées pour engager ou désengager la canule (302) de l'ensemble de sécurisation d'une canule.

11. Ensemble de sécurisation d'une canule (300) selon la revendication 1, dans lequel la plaque de verrouillage (504) est une plaque plate.
